# FASCICULE DE BREVET EUROPEEN

(11) **EP 1 151 127 B1**
(45) Date de publication et mention de la délivrance du brevet: **22.06.2005**
(21) Numéro de dépôt: 00901666.8
(22) Date de dépôt: 28.01.2000
(51) Int. Cl.: C12P 7/06

(54) **PROCEDE DE PRODUCTION D'ETHANOL AVEC APPORT FREQUENT DE LEVURE**
VERFAHREN ZUR ETHANOLHERSTELLUNG MIT KONTINUIERLICHE BEIMISCHUNG VON HEFE
METHOD FOR PRODUCING ETHANOL WITH FREQUENT INPUT OF YEAST

(30) Priorité: 04.02.1999 FR 9901297
(43) Date de publication de la demande: 07.11.2001
(73) Titulaire: Bio-Ethanol Nord Picardie, 02390 Origny-Sainte-Benoite (FR)
(72) Inventeur: ALARD, Georges, Maurice, F-02120 Guise (FR); ROUX, Philippe, Jean, F-02100 St-Quentin (FR); MOURIN, Alain, Yves, Gérard, F-02240 Ribemont (FR); BRASSEUR, Luc, Robert, F-02240 Pleine-Selve (FR)
(74) Mandataire: Eidelsberg, Victor Albert
(86) Numéro de dépôt international: PCT/FR2000/000199
(87) Numéro de publication internationale: WO 2000/046387

(56) Documents cités:
- WO-A-92/20777
- CH-A- 554 414
- FR-A- 2 697 266
- US-A- 4 419 448
- DELIA-DUPUY M L ET AL: "Contamination par les levures Brettanomyces dans les fermentations alcooliques." M.A.N. MICROBIOLOGIE ALIMENTS NUTRITION., vol. 13, 1995, pages 349-359, XP002119778 LE PLESSIS-ROBINSON., FR ISSN: 0759-0644

## Description

La présente invention se rapporte aux procédés de production d'éthanol à partir de matière première végétale amylacée. Elle s'applique particulièrement à la production d'éthanol en tant que carburant, mais aussi pour les industries chimiques, pharmaceutiques et cosmétiques et, après rectification de manière à enlever les substances aromatiques, alimentaires. L'invention prend notamment comme matière de départ un moût de blé, de maïs, d'orge, de sorgho, de seigle ou de riz.

Au WO-92-20777, on décrit un procédé de production d'éthanol comprenant les stades qui consistent à liquifier un premier ingrédient à la présence d'une alpha-amylase, à saccharifier le résidu pour obtenir un moût saccharifié et à fermenter par des levures du genre Saccharomyces pour transformer les sucres du moût saccharifié en éthanol. Ce procédé permet par exemple d'obtenir un vin d'une teneur en sucre de 0,59 g/l pour une teneur en alcool de 13,44 % en volume.

Dans l'article de DELIA-DUPUY M L et AL., M.A.N. Microbiologie Aliments Nutrition, volume 13, 1995, pages 349-359, on indique que, dans le cas d'une fermentation alcoolique industrielle, une contamination par des levures Brettanomyces diminue les vitesses et les rendements de production et change la composition analytique des vins. Ce document indique en particulier que, pendant les premiers jours de fermentation, la concentration en levure Saccharomyces est d'environ 6.10⁷ cellules/millilitre et que, lorsque les premières levures Brettanomyces apparaissent (à partir du 7^{ème} jour), on observe une chute de la population de Saccharomyces et cela est corrélé à une chute significative de la production d'alcool. Ce document conclut en prévoyant certaines règles à suivre pour empêcher le développement d'une contamination de ce genre et indique que la qualité de la biomasse doit être étroitement surveillée pendant la fermentation.

Au US-A-4 419 448, on mentionne que les procédés de production d'alcool sont empoisonnés par des microorganismes et qu'il est donc nécessaire de vider de temps en temps les fermenteurs, de les nettoyer et de recommencer la production d'alcool en introduisant des levures nouvelles.

On connaît déjà un procédé de production d'éthanol qui consiste à soumettre un moût de matière végétale amylacée à un traitement enzymatique de liquéfaction pour obtenir un moût liquéfié. On soumet ensuite le moût liquéfié à un traitement enzymatique de saccharification au moins partielle pour obtenir un moût saccharifié. L'amidon a été transformé au moins partiellement en glucose. On divise ensuite le moût saccharifié en une première partie et en une seconde partie. On dilue la première partie du moût saccharifié par un diluant et on la met en contact dans un préfermenteur avec une levure du genre Saccharomyces pour obtenir une suspension de levures. La proportion de diluant, qui est généralement de l'eau et/ou de la vinasse, est telle que le degré alcoolique de la suspension de levures soit inférieur à 6 % en volume, en sorte qu'une concentration trop élevée en alcool n'empêche pas les levures de se développer. On met ensuite cette suspension de levures en contact avec la seconde partie du moût saccharifié dans un fermenteur pendant une durée suffisante pour obtenir un vin ayant une teneur en éthanol supérieure à un seuil donné. Dans la pratique habituelle, cette durée est d'autant plus grande que la teneur en éthanol est plus grande. Lorsque la durée de saccharification est par exemple de 20 h, il faut couramment prévoir une durée de mise en contact dans le fermenteur ou durée de fermentation de 40 h pour obtenir une teneur en éthanol supérieure à 9 % avec une teneur en sucre inférieure à 1 g/l. On a déjà préconisé de ramener la durée de l'opération de saccharification à 10 h en faisant en sorte que la saccharification ait lieu également pendant la durée de fermentation qui reste maintenue à 40 h. La durée totale des opérations est alors de 50 h. Pour obtenir de l'éthanol, on distille le vin. Au cours de cette distillation, on effectue une extraction des "mauvais goûts" qui correspondent notamment aux esters, de manière à pouvoir obtenir de l'éthanol ayant moins de 500 parties d'esters par million, comme cela est exigé couramment pour l'utilisation de l'éthanol en tant que carburant. Il est nécessaire en outre d'ajouter en continu de l'acide dans le préfermenteur pour maintenir une certaine asepsie vis-à-vis des bactéries et de désinfecter les circuits à l'aide de désinfectant.

L'invention pallie ces inconvénients par un procédé de production d'éthanol de durée bien plus courte qu'avec les procédés antérieurs, qui donne directement par distillation un alcool contenant si peu d'esters qu'il n'est plus nécessaire d'en prévoir l'extraction, qui permet de supprimer toute addition d'acide au préfermenteur et de diminuer la quantité de désinfectant utilisé dans les circuits de fabrication, tout en ayant, toutes conditions égales par ailleurs, un degré alcoolique du vin plus élevé et la même teneur basse en sucres.

L'invention a pour objet un procédé de production d'éthanol qui consiste à mettre un moût de matière première végétale amylacée en contact avec une enzyme de liquéfaction pour obtenir un moût liquéfié, à mettre le moût liquéfié en contact avec une enzyme de saccharification pour obtenir un moût saccharifiée au moins partiellement, à préparer dans un préfermenteur une suspension de levures du genre Saccharomyces dans un milieu nutritif, à mettre le moût saccharifié en contact avec de la suspension de levures pour transformer en éthanol les sucres contenus dans le moût saccharifié en obtenant un vin ayant une teneur en sucres inférieure à 3 g/l, de préférence inférieure à 2 g/l et, mieux, inférieure à 1 g/l pour un degré alcoolique d'au moins 9,5 % en volume par volume et à distiller le vin pour obtenir de l'éthanol,
caractérisé en ce qu'il consiste, à un intervalle de temps tel que la concentration en micro-organismes, autres que les levures du genre Saccharomyces, dans le préfermenteur reste inférieur à un seuil donné de 10⁷ cellules par millilitre pendant l'intervalle suivant le remplacement des levures, à enlever toutes les levures présentes dans le préfermenteur, à nettoyer et à désinfecter le préfermenteur et y mettre des levures fraîches.

On a en effet constaté, d'une manière inattendue, que la durée très longue du contact de la suspension de levures avec le moût saccharifié, qui était nécessaire jusqu'ici, est due au fait qu'en relativement peu de temps, la levure du genre Saccharomyces subit une dégénérescence, une mutation et/ou une contamination par un autre micro-organisme, notamment par une levure du genre Brettanomyces bien moins active. En remplaçant toutes les levures par des levures fraîches du genre Saccharomyces avant que ne se produise ce phénomène, et notamment de préférence avant qu'il y ait 10⁷ cellules par millilitre, et mieux 10⁶ cellules par millilitre, d'un micro-organisme autre que la levure du genre Saccharomyces dans le préfermenteur, on maintient l'activité des levures de fermentation, ce qui permet de diminuer la durée de mise en contact dans le fermenteur, de ne plus avoir pratiquement d'ester lors de la distillation, d'avoir un degré alcoolique plus élevé, de supprimer la nécessité d'ajouter de l'acide dans le préfermenteur et de diminuer la quantité de désinfectant. La fraîcheur de la suspension de levures joue un rôle primordial sur la durée de l'étape de transformation des sucres en éthanol. L'apport de levures fraîches aux levures déjà contaminées ne donne une amélioration de la durée de contact nécessaire que très temporaire. Pour maintenir de manière continue la durée de contact écourtée que l'on souhaite, il faut éliminer, avant de mettre de nouvelles levures, pratiquement toutes les levures anciennes.

On peut détecter la présence de Brettanomyces dans le préfermenteur en prélevant un échantillon de la suspension de levures et en l'examinant au microscope. Alors que les Saccharomyces, et notamment Saccharomyces cerevisiae, ont une forme ovoïde, les Brettanomyces ont une forme allongée. On peut également savoir par l'expérience le délai à respecter pour l'ajout des levures fraîches et remplacer systématiquement les levures usées par des levures Saccharomyces à un intervalle de temps inférieur à 4 jours.

Suivant un mode de réalisation préféré, le procédé consiste à diluer une première partie de 10 à 30 % et de préférence de 15 à 20 % du poids du moût saccharifié pour obtenir un moût faible, le reste du moût saccharifié constituant un moût fort, à faire préfermenter le moût faible dans le préfermenteur pour obtenir un moût préfermenté et à mettre le moût fort en présence du moût préfermenté dans un fermenteur pendant une durée suffisante pour obtenir le vin.

De préférence, on apporte des levures fraîches au préfermenteur en une quantité telle que l'on obtient dans le préfermenteur une concentration au moins égale à 10⁶ cellules par millilitre environ et, de préférence, à 10⁷ cellules par millilitre.

On a obtenu, notamment pour un seuil de 9,5 % de la teneur en éthanol du vin, que la somme de la durée de la saccharification et de la durée de mise en contact dans un fermenteur soit seulement de 35 h.

Le premier stade du procédé suivant l'invention consiste à soumettre un moût de matière végétale amylacée à un traitement enzymatique de liquéfaction pour obtenir un moût liquéfié.

La matière végétale, notamment le blé, est broyée par exemple dans un broyeur à marteaux en une ou deux passes (marque PROMILL Promill-Stolz, RN 12 Serville, 28410 BU, 3000 tr/mn ou marque JACKERING Vorsterhauser Weg 46 PO BOX 1733, 59007 HAMM), plutôt dans les cas où les sons ne sont pas séparés de la farine, dans un ou plusieurs broyeurs à cylindres (où la mouture est plus homogène), plutôt dans les cas où les sons sont séparés de la farine ou tout autre type de broyeur. On peut éventuellement, dans le cas d'une amidonnerie, séparer la farine en deux qualités : une farine A destinée au procédé amidonnier, une farine B destinée au procédé éthanol. Le blé peut éventuellement être humidifié entre 18 et 25 % en poids avant broyage afin d'améliorer la séparation entre la farine et les sons.

La farine obtenue est tamisée. Le refus du tamis est recyclé vers le broyeur, de manière à ce que les plus gros grains ne dépassent pas 2,5 mm. Couramment, le pourcentage de particules supérieures à 1 mm ne doit pas dépasser 10 % du total. L'ouverture moyenne de la farine est comprise entre 0,3 et 2 mm, 0,6 constitue une valeur courante. Les sons qui sont séparés au niveau du tamisage sont soit réintroduits dans la farine, soit séparés. On obtient alors soit une farine complète soit une farine blanche.

Dans le cas d'une farine complète, la farine est alors mélangée dans un mélangeur avec une solution d'eau, de vinasse, de soude et une enzyme de liquéfaction. Cette solution est réalisée par exemple au moyen de mélangeurs statiques en ligne ou dans un bac de préparation. Le mélange farine/solution peut être réalisé soit dans une vis mélangeuse (marque PROMILL Promill-Stolz, RN 12 Serville, 28410 BU, vitesse de rotation : 700-1200 tr/mn), soit et/ou puis dans un homogénéisateur (marque : APV GAULIN, pression : environ 100 bars), soit dans un bac agité.

Dans le cas d'une séparation du gluten, on ne recycle pas les vinasses provenant de la distillerie à l'empâtage. La solution est réalisée au moyen de lait d'amidon provenant du procédé amidonnier et de farine B.

On obtient alors un moût de 25 à 35 % en poids de matières sèches. Le pourcentage de matières sèches est déterminé par la matière sèche optimale de fonctionnement des enzymes de liquéfaction et de saccharification et par un souci économique d'avoir la matière sèche la plus élevée possible afin de limiter les coûts de l'évaporation des vinasses dans la suite du procédé. La quantité de farine apportée au mélangeur est régulée soit par un doseur SCHENCK, Chemin neuf BP 17, 78240 CHAMBOURCY, soit par une bande peseuse.

Afin d'avoir une matière sèche des vinasses clarifiées (séparation de la fraction insoluble des vinasses par décantation centrifuge) la plus élevée possible (entre 4,5 et 7 % en poids), on cherche à introduire le plus de vinasses possible à l'empâtage au lieu d'apporter de l'eau extérieure. Ceci détermine la fraction de vinasses par rapport à l'eau dans l'opération d'empâtage. Les vinasses clarifiées représentent de 40 à 80 % en poids de la solution utilisée pour réaliser l'empâtage. L'eau peut être une eau de forage, une eau de procédé (condensats d'évaporation ou flegmasses), ou une eau de rivière préalablement filtrée sur un filtre à sable et/ou stérilisée par des rayonnements UV.

La température du mélange eau/vinasse est comprise entre 40°C afin de faciliter le mélange et afin de limiter la consommation d'énergie pour la liquéfaction et 70°C maximum afin de ne pas gélatiniser l'amidon pendant l'empâtage. La température des vinasses à la sortie des décanteuses centrifuges (GUINARD CENTRIFUGATION, ZI du Buxerioux, BP 69, 36002 Châteauroux ; WESTFALIA SEPARATOR, 18, avenue de l'Europe, BP 120, 02407 Château-Thierry) est comprise entre 70 et 100°C selon le procédé de distillation (la température est plus basse dans le cas d'une distillation sous vide). La farine est à température ambiante. Un échangeur à plaques ou un échangeur tubulaire ou tout type d'échangeur de chaleur doit être prévu pour réchauffer l'eau de manière à obtenir la température du mélange évoquée ci-dessus.

Selon l'enzyme de liquéfaction utilisée, le pH doit être ajusté avec de la soude à 30 ou 50 % ou tout autre agent alcalinisant. Un sel de calcium peut être employé éventuellement si l'enzyme le demande. Les enzymes utilisées sont des alpha-amylases fongiques ou bactériennes, par exemple Termamyl 120L type S, type L ou type LS de NOVO NORDISK Bioindustries S.A., 79, av. François-Arago, 92017 Nanterre Cedex, France ; la SPEZYME AA ou SPEZYME AAL de GENENCOR P.O., Box 642, Delft, Pays-Bas ; la NERVANASE ou G-ZYME G995 de RHODIA, Poleacre Lane, Wooddley, stockport, Cheshire, SK6 1PQ, Royaume-Uni). Le débit de soude est régulé au moyen d'une sonde de pH installée sur le mélange eau/vinasses avant empâteur. Le pH peut varier entre 4,5 et 8 selon les enzymes utilisées.

La liquéfaction est réalisée à une température comprise entre 50 et 100°C. Le moût empâté peut être porté à cette température soit par le biais d'une injection directe de vapeur dans le bac de liquéfaction par des tuyauteries ; soit par un jet-cooker, auquel cas le moût empâté est porté pendant quelques secondes à 100-150°C au moyen d'une injection de vapeur dans une tuyère avant d'être refroidi rapidement entre 80 et 95°C. Le débit d'enzymes peut être asservi au débit de farine.

Les bacs de liquéfaction peuvent être agités par exemple par des agitateurs de marque PMS, BP 72 91560 Crosne à deux rangées de pales pour le premier bac et une pour le deuxième, vitesse de rotation : 42 tr/mn pour le premier bac, 58 tr/mn pour le deuxième ; vitesses de rotation pouvant être comprises entre 20 et 60 tr/mn). Le temps de séjour dans ces conditions de température est compris entre 30 minutes et deux heures.

Les caractéristiques actuelles du procédé de liquéfaction dépendant de l'enzyme utilisée sont :
Température : 85-88°C
pH : 5,5-6
Matière sèche : 32 %-35 %
Temps de séjour : 1 heure
Débit d'enzyme de liquéfaction : environ 3,5 litres/heure pour une alimentation de farine de 8 tonnes/heure

Le moût ainsi liquéfié est refroidi dans des échangeurs thermiques de type classique (échangeurs à plaques ou échangeurs tubulaires) à 60°C (température : fonction des conditions optimales d'utilisation des enzymes de saccharification pouvant varier de 40°C à 70°C).

Dans certains cas, une dilution du moût liquéfié peut être effectuée avec un diluant tel que l'eau ou des vinasses de recyclage provenant de la distillerie comme citées ci-dessus.

Le moût liquéfié est mis en présence d'enzyme de type amyloglucosidase (ex : Optimax 7525 HP, Optidex L300 de la Société GENENCOR International, Box 642, 2600 AP Delft, Pays-Bas, Amg 300 L de la société Novo Nordisk Bioindustries S.A., 79, av François-Arago, 92017 Nanterre Cedex, France, la G-990 ou l'Ambazyme LE300 de RHODIA, Poleacre Lane, Wooddley, stockport, Cheshire, SK6 1PQ, Royaume-Uni) et d'enzyme déviscosante (ex : Econase CE de la Société Alko Biotechnology, SF-05200 Rajamaki, Finlande, Celluclast de la Société Novo Nordisk Bioindustries S.A., 79, av. François-Arago, 92017 Nanterre Cedex, France ; la β-Glucanase 750 L de RHODIA, Poleacre Lane, Wooddley, stockport, Cheshire, SK6 1PQ, Royaume-Uni).

Selon les enzymes de saccharification utilisées, le pH doit être ajusté avec de l'acide sulfurique à environ 96 % ou tout autre agent acidifiant. Le débit d'acide est régulé au moyen d'une sonde de pH installée en entrée de saccharification. Le pH peut varier entre 3 et 7 selon les caractéristiques optimales des enzymes utilisées.

Des enzymes ayant une activité protéase (tel que la Protéinase 200L de RHODIA, Poleacre Lane, Wooddley, stockport, Cheshire, SK6 1PQ, Royaume-Uni) et/ou pullulanase (tel que l'Ambazyme P20 de RHODIA, Poleacre Lane, Wooddley, stockport, Cheshire, SK6 1PQ, Royaume-Uni ou l'Optimax L300 de GENENCOR P.O., Box 642, Delft, Pays-Bas) peuvent également être utilisées selon les types de substrats afin respectivement de dégrader les protéines présentes dans le moût liquéfié source d'azote potentielle pour les organismes fermentaires) ou de parfaire l'hydrolyse enzymatique de l'amidon (les pullulanases ont une action spécifique au niveau de la liaison α 1-6).

Les débits des enzymes peuvent être asservis au débit de farine entrant et sont également fonction des analyses de laboratoire portant sur la concentration en glucose produit et la teneur en amidon restant ; l'objectif de cette opération est de terminer en fin de saccharification ou de fermentation (selon les procédés adoptés) avec une absence d'amidon restant dans le vin entrée distillerie. Ces débits sont donc réglés en fonction de l'activité enzymatique spécifique à chaque type d'enzyme.

Les caractéristiques actuelles du procédé de saccharification dépendantes des enzymes utilisées sont :
Température : 55-65°C
pH : 4 à 4,5
Matière sèche : 28 %-35 %
Temps de séjour : 15-20 heures (exemple 1A) : ou entre 8-13 heures (exemple 1B)
Débit d'enzyme de saccharification : environ 4,5 litres/heure pour une alimentation de farine de 8 tonnes/heure
Débit d'enzyme déviscosante : environ 1,5 litres/heure pour une alimentation de farine de 8 tonnes/heure.

La saccharification utilise cinq cuves de 90 m³ (fonction des exemples 1A et 1B).

Les 5 bacs de saccharification ont une agitation mécanique (agitateurs de marque SEW-USOCOME ou PMS BP 72 91560 Crosne, vitesse de rotation 24 tr/mn), agitation permettant une bonne homogénéisation du moût en cours de saccharification et par là même une mise en contact facilitée entre les enzymes et l'amidon à hydrolyser.

Le moût saccharifié est refroidi à 32°C (température comprise entre 30 et 34°C, ceci afin de ne pas inhiber le développement et la fermentation des levures utilisées) dans des échangeurs thermiques classiques avant d'être envoyé dans l'atelier suivant.

Deux modes de réalisation du procédé peuvent être mis en oeuvre. Ils consistent à réaliser la bioconversion totale ou partielle de l'amidon macromolécule en molécules fermentescibles de glucose. Dans le deuxième cas, (hydrolyse très partielle) la saccharification se poursuit durant l'étape de fermentation, le temps de séjour dans la saccharification étant beaucoup plus court que dans le premier cas (hydrolyse presque totale).

### Préfermentation

La préfermentation ou propagation des levures (ex : Saccharomyces cerevisiae, Saccharomyces pombe...) peut être avantageusement réalisée pour obtenir une concentration au moins égale à 10⁷ cellules par millilitre dans 4 préfermenteurs (volume des préfermenteurs : 45 m³ chacun) fonctionnant en parallèle.

Le moût saccharifié provenant de la saccharification est dilué avec de l'eau ou de la vinasse pour obtenir un moût faible (débit d'eau : 7 à 8 m³/h pour un débit de moût saccharifié de 4-5 m³/h, ceci afin d'obtenir une concentration en glucose dans le moût faible oscillant entre 50 et 90 g/l) qui est distribué dans les 4 préfermenteurs et sert de substrat de croissance aux micro-organismes. L'eau peut être une eau de forage, une eau de procédé (condensats d'évaporation, flegmasses ou eau provenant de l'amidonnerie) ou une eau de rivière préalablement filtrée sur un filtre à sable et/ou stérilisée par des rayonnements UV.

La température dans les préfermenteurs est rigoureusement contrôlée et régulée par un système de plaques de refroidissement dans lequel circule un liquide de refroidissement à l'intérieur ou à l'extérieur des cuves de préfermentation.

Toute multiplication de micro-organismes entraîne une élévation de température. Toute variation de température peut devenir inhibitrice pour la propagation des levures.

La température dans les préfermenteurs est maintenue entre 30 et 35°C.

Afin de favoriser le développement des levures, le milieu nutritif nécessaire à la multiplication de ces micro-organismes comprend
- de l'azote apporté sous formes diverses telles que l'urée, l'ammoniac, les sels d'ammonium,
- du phosphore apporté sous formes diverses telles que l'acide phosphorique, les phosphates,
- du soufre apporté sous formes diverses telles que l'acide sulfurique, les sulfates,
- de l'oxygène,
- des sucres fermentescibles,
- des minéraux essentiels si une carence quelconque est détectée.

Ces éléments nutritifs préviennent tout ralentissement de la propagation des levures.

Ces éléments nutritifs et l'oxygène sous forme d'air comprimé (ou d'eau oxygénée) sont régulièrement fournis afin de favoriser le développement des levures et non la fermentation alcoolique.

A titre d'exemple :
- Le débit d'air dans chaque préfermenteur est d'environ 30 Nm³/h.
- 5 kg de sulfate d'ammonium ayant une teneur en azote minimale de 21 % et en eau de 0,2 % maximum (HOLVOET Chimie Chaussée de Leuze 144, Leuzesesteenweg Belgique ; INTERFERT, 28, rue d'Armenonville, 92200 Neuilly sur Seine...) et 5 kg de phosphate diammonique d'une pureté de 95 % minimum et ayant une teneur en P₂O₅ comprise entre 52 et 55 % (RHODIA Chimie, 299, rue du Président Pompidou, BP 202, 59561 La Madeleine Cedex ; PRAYON France, 80-82, rue de Paris, 93804 Epinay sur Seine Cedex) sont versés toutes les heures dans chaque préfermenteur sous forme d'une solution aqueuse.

Afin d'éviter tout risque d'infection par des micro-organismes étrangers, l'installation industrielle se trouvant en plein air ; les préfermenteurs sont nettoyés et désinfectés à tour de rôle par nettoyage à l'eau filtrée de rivière puis injection de vapeur durant une durée prédéterminée de 10 minutes minimum.

Cela permet par ailleurs d'éviter les infections par des levures dites "sauvages" et la dégénérescence progressive de la souche levurienne prédominante.

Une identification de ces micro-organismes contaminants détectés sur le site a été réalisée : il s'agit de Brettanomyces bruxellensis.

Cette levure contaminante a une forme allongée caractéristique très différente de la morphologie de la Saccharomyces cerevisiae utilisée.

Des observations microscopiques quotidiennes (Microscope universel à lumière transmise de marque ZEISS à grossissement X 400) des moûts levurés ont été mises en place sur le site de fabrication et permettent de détecter instantanément l'apparition de ces levures sauvages de type Brettanomyces.

Des comptages et reconnaissances morphologiques sur boîtes de Petri viennent confirmer à posteriori les observations microscopiques.

Le milieu utilisé pour le comptage sur boîtes de Petri dénommé milieu de MALT WICKERHAM est composé de pour 2 litres de préparation :
- extrait de malt 3 g (référence Laboratoire Merck 105391)
- peptone 5 g (référence Laboratoire Merck 7212)
- extrait de levure 3 g (référence Laboratoire Merck 103753)
- glucose 10 g
- gélose nutritive 10 g (référence Laboratoire Merck 1614)

L'échantillon du moût levuré est dilué au dixième successivement dans du sérum physiologique (solution de NaCl à 9 pour mille) jusqu'à obtenir les dilutions suivantes : 10⁻⁵ ; 10⁻⁶ ; 10⁻⁷. 1 ml de ces dilutions est ensemencé en profondeur selon les techniques classiques de microbiologie avec le milieu de MALT WICKERHAM.

L'espacement de la fréquence de rajout de la souche souhaitée pour le processus fermentaire a pour conséquence une contamination prioritaire par l'organisme microbien indésirable entraînant une augmentation des temps de fermentation ainsi qu'une altération de la qualité des flegmes produits (ex : augmentation de la teneur en esters). Ce phénomène fut observé quelles que soient les variantes du présent procédé.

Un retard dans la fréquence impérative de rajout de la souche souhaitée et de remplacement des levures anciennes entraîne par ailleurs la nécessité d'accélérer les adjonctions de levures afin d'éradiquer la persistance de la contamination due au recyclage des vinasses claires, contenant des organismes contaminants, à l'empâtage.

### Procédé de remplacement des levures anciennes par des nouvelles levures

Lors de la propagation de levures fraîches, par exemple un préfermenteur sur quatre est choisi pour la réalisation de cette opération, les actions à mener sont les suivantes :

Le préfermenteur étant vidangé, le laver à l'eau puis le nettoyer à la vapeur (Par exemple : Pression = 3 bars absolus, température = 130°C) selon une durée prédéterminée 10 minutes minimum.

On peut également réaliser ce nettoyage avec de l'eau formolée (Formol 30,5% : Caldic France B.P. 722 51056 REIMS) ou tout désinfectant classique tels que :
Les familles des composés halogènes : chlore ou iode et leurs dérivés
Les agents d'oxydation (peroxyde d'hydrogène, permanganate de potassium) Les composés aminés (Bactanios 95 employé en solution de 0,2 à 0,5 %, Anios Pavé du Moulin 59260 Lilles Hellemmes).

Les acides et alcalis forts (L'acide sulfurique concentré à 96% employé à une dilution de 5 à 10%, Tessenderlo Chemie rue du Trône 130 B Bruxelles), (La lessive de soude concentrée à 30,5 %, employée à chaud à une dilution de 1 à 2 %, CLEMENT RPC Ets LOMME Rue Pelouze, BP 117 59461 LOMME cedex) (Agrobac employé à des dilutions de 2 à 7,5 % : MINOT APURA 88 rue de Marquillies 59044 LILLES cedex), (Agromousse employé à des dilutions de 5 à 7%, MINOT APURA 88 rue de Marquillies 59044 LILLES cedex). (L'Aniosteril acide désinfectant employé à des doses de 1 à 1,5% Anios Pavé du Moulin 59260 Lilles Hellemmes), (GALOR C7 employé à des doses de 1 à 5%, Anios Pavé du Moulin 59260 Lilles Hellemmes).

Les aldéhydes et tensioactifs (Anios W4 employé à 0,5 % en pulvérisation temps de contact 5 à 10 minutes ou en circulation à 0,4 % temps de contact 20 à 30 minutes, Anios Pavé du Moulin 59260 Lilles Hellemmes).

Les pourcentages indiqués ci-dessus sont exprimés en poids.

La procédure de nettoyage ― désinfection peut comporter 5 phases :
- prélavage ou pré-nettoyage : élimination mécanique des souillures grossières par jet d'eau,
- nettoyage : élimination du reste des souillures à l'aide d'une solution de nettoyage,
- rinçage : élimination de la solution de nettoyage dans laquelle les souillures ont été dispersées,
- désinfection : destruction chimique de la biocontamination de surface à l'aide d'une solution désinfectante,
- rinçage final: élimination de la solution désinfectante résiduelle.

Une combinaison des opérations citées ci-dessus permet dans tous les cas d'aboutir à une désinfection suffisante du préfermenteur destiné à recevoir les levures fraîches sans contamination avec les anciennes levures.

Le préfermenteur est rempli à un tiers environ de sa capacité avec du moût faible un peu plus dilué que la normale.

On régule rigoureusement la température dans le préfermenteur (inférieure à 34°C).

Les 300 kg de levures fraîches à environ 32% en poids de matières sèches sont ajoutés dans le préfermenteur.

Un apport en sels nutritifs et en air est réalisé et régulé.

L'alimentation en moût faible est poursuivie tout en contrôlant la densité et la température dans le préfermenteur.

Ce dernier, une fois rempli, est mis en communication successivement avec les autres préfermenteurs préalablement vidés, nettoyés et stérilisés à la vapeur ou chimiquement de façon à ne pas contaminer les nouvelles levures par les anciennes.

On alimente ainsi progressivement les 4 préfermenteurs avec les nouvelles levures.

### Fermentation

La fermentation alcoolique peut être réalisée à partir du moût saccharifié venant de la saccharification à un débit de 14 à 22 m³/h jusqu'à ce que la teneur en sucres du vin obtenu soit inférieure à 3 g/l et, de préférence, à 2 g/l et mieux à 1 g/l, pour un degré alcoolique du vin d'au moins 9,5% en volume.

Deux types de fermentation peuvent être réalisés :

La fermentation «batch » ou discontinue, procédé par lequel chaque fermenteur fonctionne individuellement, c'est-à-dire que la fermentation est menée à son terme dans chaque fermenteur.

La fermentation continue, procédé par lequel les fermenteurs travaillent en cascade, c'est-à-dire que la fermentation n'est que partielle dans chaque fermenteur jusqu'au dernier où la fermentation est achevée.

### La fermentation «batch » ou discontinue

Chaque fermenteur est ensemencé altemativement par le moût levuré provenant des préfermenteurs.

L'envoi du moût préfermenté est réalisé selon le procédé suivant :
- 1 préfermenteur (volume : 45 m³) est vidé dans sa totalité vers le fermenteur.
- les 3 autres sont transférés partiellement en parallèle pour complémenter le "pied de levures" dans les fermenteurs.

Le volume total du moût levuré transféré correspond à environ 30-70 % du volume du fermenteur. La température dans les fermenteurs est maintenue entre 30 et 35°C.

La fermentation utilise deux cuves de 90 m³ et 6 cuves de 180 m³. Le moût fermenté est ensuite transféré dans les bacs à vins avant d'alimenter l'atelier de distillerie.

### La fermentation continue :

L'alimentation des fermenteurs en moût levuré et moût saccharifié ne se fait plus alternativement comme décrit ci-dessus mais selon le procédé suivant :

On alimente en continu en moût levuré et en moût saccharifié le ou les deux ou les trois premiers fermenteurs, appelés fermenteurs de tête, les fermenteurs suivants étant nommés fermenteurs de chute.

Le nombre de fermenteurs de tête et la répartition des alimentations en moûts dépendent des volumes des fermenteurs.

Les débits d'alimentation des fermenteurs de tête peuvent être les suivants :
Moût levuré : 9 à 16 m³/h
Moût saccharifié : 15 à 25 m³/h
La température dans les fermenteurs est maintenue entre 30 et 35°C.

La fermentation se poursuit ensuite en cascade dans chaque fermenteur jusqu'à ce que la teneur en sucres du vin obtenu soit inférieure à 3g/l et, de préférence à 2 g/l, et mieux à 1 g/l, pour un degré alcoolique du vin d'au moins 9,5% en volume, le moût fermenté du dernier fermenteur est alors envoyé toujours en continu dans les bacs à vin, avant d'alimenter l'atelier de distillerie.

Dans ce type de fermentation continue, la durée de fermentation est calculée en divisant le volume de remplissage de moûts en fermentation dans l'ensemble des fermenteurs par le débit d'alimentation en vin des colonnes de distillerie. On obtient en fermentation continue les mêmes temps de fermentation qu'en fermentation discontinue.

On détermine le degré alcoolique des vins par voie enzymatique à l'aide de l'analyseur biochimique YSI 2700 SELECT (ROUCAIRE, 2 av. du Pacifique BP 78 Les Ulis 91493 Courtaboeuf cedex). On effectue une dilution au 50^{ème} avec de l'eau déminéralisée de l'échantillon de vin. On filtre cet échantillon et on le passe ensuite dans l'analyseur biochimique YSI 2700 SELECT qui donne automatiquement la teneur en éthanol en g/l. Pour obtenir le résultat en degré alcoolique (% volume par volume), il suffit de diviser cette valeur par 7.88 après avoir pris en compte le facteur de dilution.

On détermine la teneur en glucose restant par vole enzymatique à l'aide de l'analyseur biochimique YSI 2300 STAT PLUS (ROUCAIRE, 2 av. du Pacifique BP 78 Les Ulis 91493 Courtaboeuf cedex). On effectue les dilutions nécessaires des échantillons selon la teneur supposée en glucose restant. On filtre l'échantillon et on le passe ensuite dans l'analyseur biochimique YSI 2300 STAT PLUS qui donne une teneur en glucose restant exprimée en mg/dl. Pour l'obtenir en g/l, il suffit de multiplier le résultat obtenu par 100 après avoir pris en compte le facteur de dilution éventuel.

### Production de flegmes (alcool brut)

(La) ou les colonne(s) distillatrice(s) (Fournisseurs : KREBS-SPEICHIM, 14 rue Hoche, 92800 PUTEAUX, JAAKKO-PÖYRY, Garden Part-Dieu, 65 Bd Vivier Merle 69482 LYON CEDEX 03) qui peuvent fonctionner en parallèle ou série (double effet) sous vide ou sous pression, sont chauffées par les vapeurs provenant, par exemple, de la concentration de vinasses (coproduits de la distillerie) afin d'améliorer les performances thermiques de l'unité soit en injection directe, soit par l'intermédiaire de bouilleurs, soit par thermocompression. Ces vapeurs chauffent également les colonnes de Lutter.

Le moût fermenté ou vin provenant.de la fermentation, après passage dans un échangeur thermique, alimente parallèlement les colonnes distillatrices. Les vapeurs d'alcool des colonnes sont condensées dans des échangeurs thermiques.

Les vinasses sortant en pied des colonnes sont envoyées à l'atelier de séparation des drêches pour être clarifiées (séparation des matières solubles des insolubles) avant d'être envoyées vers l'atelier de concentration de vinasses.

L'alcool ou flegme est concentré à 90-96 % volume (selon les contraintes d'investissement) dans la ou les colonnes distillatrices puis refroidi dans des échangeurs avant stockage. Une extraction des impuretés volatiles est également réalisée sur la ou les colonnes de distillation afin d'améliorer la qualité des flegmes (fonction de la teneur en esters recherchée). Ces mauvais goûts extraits et moins valorisables sont stockés dans une cuve différente.

Les exemples suivants illustrent l'invention.

### Exemple 1A : Durée de saccharification = 15-20 heures (essai avec du blé)

Toutes les cuves de saccharification sont remplies, ceci afin d'obtenir une hydrolyse enzymatique presque complète des molécules d'amidon en sucres fermentescibles. L'allure de l'atelier est d'environ 24 m³/h de moût saccharifié.

On remplace les levures anciennes à une fréquence inférieure à 4 jours par 300 kg de levures (ex : Saccharomyces cerevisiae) pressées à 32 % de matières sèches; chaque gramme de produit contient environ 10 milliards de cellules vivantes, ceci pour un moût faible à une concentration de 50-90 g/l en glucose fonction de l'allure de l'atelier (Débit Moût Faible : 12-13 m³/h). L'utilisation de levures lyophilisées ou de crèmes concentrée commerciale de levures (Saccharomyces cerevisiae ou pombe) permet également d'aboutir au même résultat (Fournisseurs de levures : LALLEMAND S.A., Complexe scientifique Rangeuil, Hall Gilbert Durand 3, BP 4412, 31405 Toulouse Cedex 4 ; LESAFFRE, 41, rue Etienne Marcin, 75001 Paris...)

On a ainsi observé un temps de fermentation de 18 à 24 heures (moyenne : 20 heures) avec un degré alcoolique du vin obtenu supérieur à 9,5 % minimum volume et une concentration en sucres restants inférieure à 1 g/l contre 35 à 45 heures avec des concentrations en sucres restants pouvant atteindre 20-30 g/l brutalement et de manière ponctuelle dans les conditions industrielles généralement pratiquées (les temps classiques de fermentation sur substrat amylacé préconisés par les constructeurs ou recensés dans la littérature sont de l'ordre de 40 à 60 heures).

La durée de fermentation est calculée comme suit : on additionne le temps de remplissage en moût saccharifié du fermenteur avec la durée de chute (c'est-à-dire soit jusqu'à l'obtention d'une teneur en glucose résiduel voisine de 0 g/l, soit jusqu'à l'envoi du vin en distillation, si la teneur en glucose résiduel voisine de 0 g/l ne peut être obtenue).

On a également obtenu une teneur en esters de l'alcool brut (flegmes) produit après simple distillation inférieure à 300 ppm sans extraction d'impuretés volatiles (soit une réduction de plus de 50 % par rapport à la teneur en esters généralement obtenue).

### Exemple 1B : Durée de saccharification = environ 8-13 heures (essai avec du blé)

Certaines cuves de saccharification sont by-passées, ceci afin d'obtenir une hydrolyse enzymatique très partielle des molécules d'amidon en sucres fermentescibles. L'allure de l'atelier est d'environ 24 m³/h de moût saccharifié.

On remplace les levures anciennes à une même fréquence et selon une même quantité par des levures (ex : Saccharomyces cerevisiae) pressées à 32 % de matières sèches.

On a ainsi observé un temps de fermentation de 22 à 30 heures (moyenne : 25 heures) avec un degré alcoolique du vin obtenu supérieur à 9,5 % minimum volume et une concentration en sucres restants toujours inférieure à 1 g/l contre 35 à 45 heures avec des concentrations en sucres restants pouvant atteindre 20-30 g/l brutalement et de manière ponctuelle dans les conditions industrielles généralement pratiquées (les temps classiques de fermentation sur substrat amylacé préconisés par les constructeurs ou recensés dans la littérature sont de l'ordre de 40 à 60 heures).

On a obtenu pareillement une teneur en esters de l'alcool brut (flegmes) produit après simple distillation inférieure à 300 ppm sans extraction d'impuretés volatiles (soit une réduction de plus de 50 % par rapport à la teneur en esters généralement obtenue).

### Exemple 2A : essai avec du maïs

Du maïs (amidon = 61-78%, protéines = 6-12%), utilisé en tant que substrat amylacé, a suivi le procédé industriel tel que décrit à l'exemple 1A. L'allure de l'atelier est d'environ 20-24 m³/h de moût saccharifié.

On continue à remplacer à une même fréquence et selon une même quantité des levures (ex : Saccharomyces cerevisiae) pressées à 32% de matières sèches.

On a ainsi observé un temps de fermentation de 18 à 24 heures avec un degré alcoolique du vin obtenu supérieur à 9,5 % minimum volume et une concentration en sucres restants toujour inférieure à 1 g/l.

On a obtenu pareillement une teneur en esters de l'alcool brut (flegmes) produit après simple distillation inférieure à 300 ppm sans extraction d'impuretés volatiles (soit une réduction de plus de 50% par rapport à la teneur en esters généralement obtenue).

### Exemple 2B : essai avec de l'orge

L'orge (amidon = 65-75%, protéines = 8-15%); utilisé en tant que substrat amylacé a suivi le procédé industriel tel que décrit à l'exemple 1A. L'allure de l'atelier est d'environ 20-24 m3/h de moût saccharifié.

On continue à remplacer à une même fréquence et selon une même quantité des levures (ex : Saccharomyces cerevisiae) pressées à 32% de matières séchées.

On a ainsi observé un temps de fermentation de 18 à 24 heures avec un degré alcoolique du vin obtenu supérieur à 9,5% minimum volume et une concentration en sucres restants toujours inférieure à 1 g/l.

On a obtenu pareillement une teneur en esters de l'alcool brut (flegmes) produit après simple distillation inférieure à 300 ppm sans extraction d'impuretés volatiles (soit une réduction de plus de 50% par rapport à la teneur en esters généralement obtenue).

### Exemple comparatif : essai de réensemencement partiel en levures fraîches

On effectue des essais de réensemencements partiels des préfermenteurs avec des levures fraîches de Saccharomyces cerevisiae.

Toutes les cuves de saccharification sont remplies, ceci afin d'obtenir une hydrolyse enzymatique la plus complète possible des molécules d'amidon en sucres fermentescibles (semblable à l'exemple 1A). L'allure de l'atelier est d'environ 24m³/h de moût saccharifié.

On apporte à une fréquence toujours inférieure à 4 jours 200 Kg de levures (Saccharomyces cerevisiae) pressées à 32% de matières sèches ; ceci pour un moût faible à une concentration de 50-90 g/l en glucose fonction de l'allure de l'atelier (Débit moût faible = 12-13 m³/h).

Les levures ne sont plus rajoutées, comme dans l'exemple 1A, dans un préfermenteur ayant été lavé et nettoyé au préalable, mais les 200 kg sont répartis de manière homogène dans les 4 préfermenteurs (50 kg dans chaque préfermenteur) dans lesquels il reste des anciennes levures contaminées (environ 25 m3 de moût dans chaque préfermenteur).

On n'obtient pas les résultats décrits à l'exemple 1 A précédent et on n'observe pas d'amélioration des temps de fermentation ni de la qualité des flegmes distillés : les temps de fermentation sont restés de l'ordre de 35 à 45 heures avec des concentrations en sucres restants pouvant atteindre 20-30 g/l brutalement et de manière ponctuelle et la teneur en esters supérieure à 300 ppm sans extraction d'impuretés volatiles.

## Revendications

1. Procédé de production d'éthanol qui consiste à mettre un moût de matière première végétale amylacée en contact avec une enzyme de liquéfaction pour obtenir un moût liquéfié, à mettre le moût liquéfié en contact avec une enzyme de saccharification pour obtenir un moût saccharifiée au moins partiellement, à préparer dans un préfermenteur une suspension de levures du genre Saccharomyces dans un milieu nutritif, à mettre le moût saccharifié en contact avec de la suspension de levures pour transformer en éthanol les sucres contenus dans le moût saccharifié en obtenant un vin ayant une teneur en sucres inférieure à 3 g/l, de préférence inférieure à 2 g/l et, mieux, inférieure à 1 g/l pour un degré alcoolique d'au moins 9,5 % en volume par volume et à distiller le vin pour obtenir de l'éthanol,
**caractérisé en ce qu'**il consiste, à un intervalle de temps tel que la concentration en micro-organismes, autres que les levures du genre Saccharomyces, dans le préfermenteur reste inférieur à un seuil donné de 10⁷ cellules par millilitre pendant l'intervalle suivant le remplacement des levures, à enlever toutes les levures présentes dans le préfermenteur, à nettoyer et à désinfecter le préfermenteur et y mettre des levures fraîches.

2. Procédé suivant la revendication 1, **caractérisé en ce que** l'enzyme de liquéfaction est une alpha-amylase et l'enzyme de saccharification est une enzyme ayant une activité protéase, pullulanase et/ou amyloglucosidase.

3. Procédé suivant la revendication 1, **caractérisé en ce que** le seuil est inférieur à 10⁶ cellules par millilitre.

4. Procédé suivant l'une des revendications 1 à 3, **caractérisé en ce qu'**il consiste à remplacer toutes les levures dans le préfermenteur par des levures fraîches dès qu'il apparaît au microscope un micro-organisme de forme allongée.

5. Procédé suivant l'une des revendications 1 à 3, **caractérisé en ce qu'**il consiste à remplacer toutes les levures dans le préfermenteur par des levures fraîches à un intervalle de temps inférieur à 4 jours.

6. Procédé suivant l'une des revendications précédentes, **caractérisé en ce qu'**il consiste à remplacer toutes les levures du préfermenteur par des levures fraîches en une quantité telle que l'on obtient dans le préfermenteur une concentration au moins égale à 10⁶ cellules par millilitre et, de préférence, à 10⁷ cellules par millilitre.

7. Procédé suivant l'une des revendications précédentes, **caractérisé en ce qu'**il consiste à diluer une première partie de 10 à 30 % et de préférence de 15 à 20 % du poids du moût saccharifié pour obtenir un moût faible, de concentration en moût inférieure à celle du moût saccharifié, le reste du moût saccharifié constituant un moût fort, à faire préfermenter le moût faible dans le préfermenteur pour obtenir un moût préfermenté et à mettre le moût fort en présence du moût préfermenté dans un fermenteur pendant une durée suffisante pour obtenir du vin.

8. Procédé suivant l'une des revendications précédentes, **caractérisé en ce qu'**il consiste à mettre le moût saccharifié en contact avec la suspension de levures à une température de 30 à 35°C.

9. Procédé suivant l'une des revendications précédentes, **caractérisé en ce que** la matière végétale est du blé.

10. Procédé suivant l'une des revendications 1 à 8, **caractérisé en ce que** la matière végétale est du maïs, de l'orge, du riz, du seigle ou du sorgho.

## Patentansprüche

1. Verfahren zur Herstellung von Ethanol, welches aus dem Zusammenbringen eines Mosts aus stärkehaltiger pflanzlicher Primärsubstanz mit einem verflüssigenden Enzym, um einen verflüssigten Most zu erhalten, aus dem Zusammenbringen des verflüssigten Mosts mit einem zuckerbildenden Enzym, um einen zumindest teilweise verzuckerten Most zu erhalten, dem Herstellen einer Suspension von Hefen der Gattung Saccharomyces in einem Nährmedium in einem Vorfermenter, dem Zusammenbringen des verzuckerten Mosts mit der Suspension von Hefen, um die in dem verzuckerten Most enthaltenen Zucker in Ethanol umzuwandeln, wobei ein Wein erhalten wird, der einen Zuckergehalt von weniger als 3 g/l, vorzugsweise weniger als 2 g/l und noch besser weniger als 1 g/l für einen Alkoholgehalt von mindestens 9,5 Volumen-%, bezogen auf das Volumen, aufweist, und dem Destillieren des Weins besteht, um den Ethanol zu erhalten, **dadurch gekennzeichnet, dass** es in einem derartigen Zeitraum, dass die Konzentration an anderen Mikroorganismen als Hefen der Gattung Saccharomyces im Vorfermenter während des Zeitraums, der auf das Ersetzen der Hefen folgt, kleiner als eine Schwelle verbleibt, die durch 10⁷ Zellen pro Milliliter gegeben ist, das Entnehmen aller im Vorfermenter vorhandenen Hefen, das Waschen und das Desinfizieren des Vorfermenters und das Hineingeben frischer Hefen umfasst.

2. Verfahren nach Anspruch 1, **dadurch gekennzeichnet, dass** das verflüssigende Enzym eine Alpha-Amylase ist und das zuckerbildende Enzym ein Enzym ist, das eine Protease-, Pullullanase- und/oder Amyloglucosidase-Aktivität besitzt.

3. Verfahren nach Anspruch 1, **dadurch gekennzeichnet, dass** die Schwelle kleiner als 10⁶ Zellen pro Milliliter ist.

4. Verfahren nach einem der Ansprüche 1 bis 3, **dadurch gekennzeichnet, dass** es aus dem Ersetzen aller Hefen im Vorfermenter durch frische Hefen besteht, sobald unter dem Mikroskop ein Mikroorganismus mit länglicher Form erscheint.

5. Verfahren nach einem der Ansprüche 1 bis 3, **dadurch gekennzeichnet, dass** es aus dem Ersetzen aller Hefen im Vorfermenter durch frische Hefen in einem Zeitraum von weniger als 4 Tagen besteht.

6. Verfahren nach einem der vorhergehenden Ansprüche, **dadurch gekennzeichnet, dass** es aus dem Ersetzen aller Hefen des Vorfermenters durch frische Hefen in einer derartigen Menge besteht, dass im Vorfermenter eine Konzentration von mindestens 10⁶ Zellen pro Milliliter und vorzugsweise von 10⁷ Zellen pro Milliliter erhalten wird.

7. Verfahren nach einem der vorhergehenden Ansprüche, **dadurch gekennzeichnet, dass** es aus dem Verdünnen eines ersten Teils von 10 bis 30 Gew.-% und vorzugsweise von 15 bis 20 Gew.-% des verzuckerten Mosts, um einen schwachen Most mit einer kleineren Konzentration an Most als der verzuckerte Most zu erhalten, wobei der Rest des verzuckerten Mosts einen starken Most bildet, dem Vorfermentieren des schwachen Mosts im Vorfermenter, um einen vorfermentierten Most zu erhalten, und dem Zusammenbringen des starken Mosts mit dem vorfermentierten Most in einem Fermenter für einen ausreichenden Zeitraum, dass Wein erhalten wird, besteht.

8. Verfahren nach einem der vorhergehenden Ansprüche, **dadurch gekennzeichnet, dass** es aus dem Zusammenbringen des verzuckerten Mosts mit der Suspension von Hefen bei einer Temperatur von 30 bis 35°C besteht.

9. Verfahren nach einem der vorhergehenden Ansprüche, **dadurch gekennzeichnet, dass** die pflanzliche Substanz Getreide ist.

10. Verfahren nach einem der Ansprüche 1 bis 8, **dadurch gekennzeichnet, dass** die pflanzliche Substanz Mais, Gerste, Reis, Roggen oder Hirse ist.

## Claims

1. Process for producing ethanol which comprises placing a starchy plant raw material must in contact with a liquefaction enzyme in order to obtain a liquefied must, placing the liquefied must in contact with a saccharification enzyme in order to obtain an at least partially saccharified must, preparing in a prefermenter a suspension of yeasts of the genus Saccharomyces in a nutrient medium, placing the saccharified must in contact with a yeast suspension to convert the sugars contained in the saccharified must into ethanol, giving a wine with a sugar content of less than 3 g/l, preferably less than 2 g/l and better still less than 1 g/l for an alcoholic degree of at least 9.5% on a volume-for-volume basis, and distilling the wine in order to obtain ethanol,
**characterised in that** it comprises, at a time interval such that the concentration of microorganisms, other than the yeasts of the genus Saccharomyces, in the prefermenter remains less than a given threshold of 10⁷ cells per millilitre during the interval following the replacement of the yeasts, removing all the yeasts present in the prefermenter, cleaning and disinfecting the prefermenter and replacing them therein with fresh yeasts.

2. Process according to claim 1, **characterised in that** the liquefaction enzyme is an alpha-amylase and the saccharification enzyme is an enzyme having a protease activity, pullulanase and/or amyloglucosidase.

3. Process according to Claim 1, **characterised in that** the threshold is less than 10⁶ cells per millilitre.

4. Process according to one of Claims 1 to 3, **characterised in that** it comprises replacing all the yeasts in the prefermenter with fresh yeasts as soon as a microorganism of elongate shape is observed by microscope.

5. Process according to one of Claims 1 to 3, **characterised in that** it comprises replacing all the yeasts in the prefermenter with fresh yeasts at a time interval of less than 4 days.

6. Process according to one of the preceding claims, **characterised in that** it comprises replacing all the yeasts in the prefermenter with fresh yeasts in an amount such that a concentration at least equal to 10⁶ cells per millilitre and preferably 10⁷ cells per millilitre is obtained in the prefermenter.

7. Process according to one of the preceding claims, **characterised in that** it comprises diluting a first portion of 10 to 30% and preferably 15 to 20% of the weight of the saccharified must in order to obtain a weak must, of a must concentration less than that of the saccharified must, the rest of the saccharified must constituting a strong must, prefermenting the weak must in the prefermenter to obtain a prefermented must and placing the strong must in the presence of the prefermented must in a fermenter for a time which is sufficient to obtain wine.

8. Process according to one of the preceding claims, **characterised in that** it comprises placing the saccharified must in contact with the yeast suspension at a temperature of 30 to 35°C.

9. Process according to one of the preceding claims, **characterised in that** the plant material is wheat.

10. Process according to one of Claims 1 to 8, **characterised in that** the plant material is corn, barley, rice, rye or sorghum.
